# EUROPEAN PATENT SPECIFICATION

(11) **EP 3 041 562 B1**
(45) Date of publication and mention of the grant of the patent: **24.04.2019**
(21) Application number: 14842863.4
(22) Date of filing: 02.09.2014
(51) Int. Cl.: A61M 25/02, A61M 15/08, A61J 15/00

(54) **APPARATUS FOR HOLDING A TUBE AND ASSOCIATED METHOD OF USE**
VORRICHTUNG ZUM HALTEN EINES ROHRES UND ZUGEHÖRIGES VERFAHREN ZUR VERWENDUNG
APPAREIL DE SUPPORT DE SONDE ET PROCÉDÉ D'UTILISATION ASSOCIÉ

(30) Priority: 04.09.2013 AU 2013903386
(43) Date of publication of application: 13.07.2016
(73) Proprietor: Geosits, Jessica Anne, Singleton, New South Wales 2330 (AU)
(72) Inventor: Geosits, Jessica Anne, Singleton, New South Wales 2330 (AU)
(74) Representative: Piotrowicz, Pawel Jan Andrzej
(86) International application number: PCT/AU2014/050205
(87) International publication number: WO 2015/031953

(56) References cited:
- WO-A1-2006/120623
- US-A- 4 336 806
- US-A- 4 480 639
- US-A- 4 660 555
- US-A- 4 742 824
- US-A- 4 742 824
- US-A- 5 681 290
- US-A1- 2006 081 256
- US-A1- 2010 292 649

## Description

### Technical Field

The invention relates to an apparatus for holding a tube and, in particular, an apparatus in the form of a tube holder for securing a nasal gastric tube on a face of a user. The invention also relates to a method of fitting or securing a tube such as a nasal gastric tube to the face of a user with a tube holder.

### Background

There are a number of medical conditions that require patients or users to require a tube fitted into the user's nose or mouth. These tubes may be, for example, oxygen tubes, oral gastric tubes or nasal gastric tubes. Such tubes are required to be secured in place and are often attached directly to the face of the user with an adhesive strip or tape.

One specific example of a tube, which is required to be fixed in use to the face of a user, is a nasal gastric tube that is fitted to the nose of the user and proceeds into the stomach of the user for feeding. The nasal gastric tube is typically fitted to the user for extended periods of time. The nasal gastric tube may then need to be adjusted in length or removed.

Currently the method to adhere and secure a nasal gastric tube to the face of a user include: Applying a layer of Duoderm™ to the user's cheek to protect the sensitive skin; Inserting the nasal gastric tube into the user's nasal passage to the correct insertion measurement; Placing a piece of tape onto the nasal gastric tube to secure the correct insertion measurement to the user's face; and checking the tube is in correct position by drawing back with a syringe on the tube to ensure that the tube is correctly placed into the user's stomach; and cutting two more pieces of tape and secure the nasal gastric tube in place on the user's face.

During the above method the user, which may be an infant or child, becomes uncomfortable and agitated. This is further exacerbated as, typically, the tape on the user's face must be changed at least two times each week as it soils and becomes ineffective and lifts up from the user's face. Also there has to be two pieces of tape on different angles to shape to the face of the user and ensure that the user does not easily dislodge the tape.

A problem with the current tape based methods is that the user, which may be an infant or child, may attempt to dislodge the tape and the tube. Another problem with the above method and use of tape is the need to regularly change the tape. Another problem is that the tube is required to be held in place whist the tape is applied, this typically requires two people to fit the tube with the first person holding the tube whilst the second person fits the tape. Other problems include the need to apply and remove multiple pieces of tape which each need to be cut to a particular length.

The invention disclosed herein seeks to overcome one or more of the above identified problems or at least provide a useful or more favourable alternative.

US 2010/292649 A1 describes a device for securing medical tubing which is an attachment for use with medical tubing, such as a nasal cannula, nasogastric/orogastric tubes or the like. The device for securing medical tubing includes a base having opposed inner and outer surfaces. Laterally opposed peripheral edges of the first surface of the base are releasably secured to one another to form a channel for receiving and releasably securing a portion of the medical tubing. The outer surface of the base is divided into an attachment portion adapted for releasable attachment to the patient's skin, and an outwardly facing portion.

US 2006/081256 describes an apparatus and a method for stabilizing at least one medical device such as a tracheal or gastric tube entering or covering at least one facial cavity such as the mouth and/or nose, taking advantage of a stabilization stent to secure to a patient's face the tracheal or gastric tube without the need to apply tape to the face. In one form, the invention comprises a transverse stent comprising a superior border opposed to an inferior border, an inner surface and an outer surface. A first facial interface is attached to a first terminal end of the transverse stent, and positioned to adhesively adhere and mechanically clasp to a first side of a patient's face. A second facial interface is attached to a second terminal end of the transverse stent and positioned to adhesively adhere and mechanically clasp to a second side of a patient's face. A docking platform attached to the outer surface of the transverse stent supports the medical device.

### Summary

In accordance with a first main embodiment there is provided, an apparatus, as disclosed in claim 1, for securing a nasal gastric tube to a face of a user between an ear of the user and a nose of the user in a fitted condition, the apparatus including an elongate body having a securing side adapted to affix to the face of the user and a tube locating side including a locator arranged to receive and locate a received portion of the tube substantially inline with the elongate body between the ear and nose of the user. The apparatus may also further include a cover coupled to the elongate body so as to be moveable between an open condition in which the received portion of the tube is receivable by the locator and a closed condition in which the received portion of the tube is concealed by the cover. The elongate body includes a first end oriented toward the nose in the fitted condition and an opposing second end (132) oriented toward the ear in the fitted condition, and wherein the elongate body is arranged to narrow toward the first end so as to provide a nose fitting portion shaped to at least partially complement the shape of the nose proximate a nostril thereof so as to allow positioning of the elongate body at least partially below and adjacent to the nostril.

In another aspect, the locator is arranged to temporarily movably hold the received portion of the tube in the open condition so as to allow for moveable adjustment of the tube prior to the cover being moved to the closed condition.

In one aspect, the elongate body is dimensioned to extend at least half way between the nose and the ear of the user. In yet another aspect, the locating side of the elongate body is substantially free of adhesive.

In one aspect, the elongate body is dimensioned to extend at least half way between the nose and the ear of the user.

In yet another aspect, the elongate body includes a first end oriented toward the nose in the fitted condition and an opposing second end oriented toward the ear in the fitted condition, and wherein the elongate body tapers toward the first end so as to provide a nose fitting portion which allows positioning of the elongate body at least partially below and adjacent to a nostril of the nose of the user.

In yet another aspect, wherein the elongate body is arranged to narrow or tapers toward the second end so as to provide a second nose fitting portion so as to allow the tube holder to be fitted to the face in a right-hand or a left-hand orientation.

In yet another aspect, the locator is provided in the form of a channel shaped to receive and locate the received portion of the tube, the channel extending lengthwise at least partially between the first end and the second end of the elongate body.

In yet another aspect, the channel terminates short of the first end and is arranged so as to allow the nasal gastric tube to pass across the nose-fitting portion to the nostril whilst the first end of the elongate body continues to extend at least partially below the nostril.

In yet another aspect, the cover is shaped to substantially match the shape of the elongate body and has a corresponding nose fitting portion arranged to align with the nose fitting portion of the elongate body in the closed condition.

In yet another aspect, the elongate body includes first and second opposing sides extending between the first and second ends, and wherein the cover includes an attached side coupled to the first side of the elongate body and an opposing free end side which substantially meets with the second side of the elongate body in the closed condition.

In yet another aspect, the second side of the elongate body includes a securing tab which is arranged to fold at least partially over the cover in the closed condition thereby securing the cover in the closed condition.

In yet another aspect, the channel is defined by two elongate members coupled to the locating face of the elongate body, the two elongate members being arranged parallel and spaced apart from one another.

In yet another aspect, the base of the channel is provided by the locating side of the elongate body such that in the fitted condition the tube lies substantially along the locating side.

In yet another aspect, the elongate members are formed from a flexible foam material.

In yet another aspect, the cover is at least one of clear or translucent so as to allow visual inspection of the tube beneath the cover.

In yet another aspect, the cover includes an inner securing surface and an outer surface, the inner securing surface being adapted to affix to the locating face of the elongate body in the closed condition and the outer surface being substantially smooth.

In yet another aspect, the cover is at least one of a urethane or polyurethane.

In yet another aspect, the cover and elongate body are formed from a unitary material being at least one of a urethane or polyurethane.

In yet another aspect, the securing side of the elongate body includes a hydrocolloid substance adapted to adhere to the skin.

In yet another aspect, the locating side of the body includes at least one of silicone, urethane or polyurethane material.

The elongated body may include a first end oriented toward the nose in the fitted condition and an opposing second end oriented toward the ear in the fitted condition, and wherein the elongate body is arranged to narrow toward the first end so as to provide a nose fitting portion shaped to allow positioning of the elongate body at least partially below and adjacent to a nostril of the nose of the user.

In accordance with a second main embodiment there is provided, a method, as disclosed in claim 14, of retaining a tube using a tube holder on a face of a user between a nose of the user and an ear of the user, the method including the steps of: affixing a securing side of an elongate body of the tube holder such that the elongate body is substantially aligned between the nose and the ear; and locating a received portion of the tube using a locator coupled to the elongate body and adapted to receive the received portion such that the received portion of the tube is substantially inline with the elongate body. The method further includes the step of: moving a cover coupled to the elongate body from an open condition to a closed condition to cover the tube when the tube is received by the locator. The method further includes the step of positioning a nose fitting portion of the tube holder at least partially below and laterally adjacent to a nostril of the nose of the user, the nose fitting portion shaped to at least partially complement the shape of the nose proximate the nostril thereof; and fitting the tube to the locator so as to extend from the locator across the nose fitting portion and into the nostril of the nose of the user.

### Brief Description of the Figures

The invention is described, by way of non-limiting example only, by reference to the accompanying figures, in which;
Figure 1 is a diagrammatic view illustrating a user fitted with a first example of an apparatus in the form of a tube holder;
Figure 2 is a perspective view illustrating the general structure of the tube holder of Figure 1;
Figure 3 is a perspective exploded parts view of the structure of a nasal gastric tube holder;
Figures 4 and 5 are illustrative perspective views of the correct relative positioning of the nasal gastric feed tube to the nasal gastric tube holder to allow correct positioning on the face and correct insertion depth of the gastric tube into the user's stomach when affixed to the patient;
Figure 6 is a perspective view illustrating a second example of the tube holder in an open condition with a tube fitted to the tube holder;
Figure 7 is a perspective view illustrating the second example of the tube holder closed condition with the tube fitted to the tube holder;
Figure 8 is a perspective view illustrating the second example of the tube holder fitted to the face of a user between the nose and the ear;
Figure 9 is a perspective view illustrating the second example of the tube holder being fitted with the tube and being moved to the closed condition;
Figure 10 is a perspective view illustrating the second example of the tube holder being fitted with the tube and being partially moved to the closed condition to cover the tube;
Figure 10a is a cross section view illustrating the second example of the tube holder as shown in Figure 6.
Figure 11 is a perspective view illustrating a third example of the tube holder in open condition with the tube fitted to the tube holder;
Figure 12 is a perspective view illustrating the third example of the tube holder being fitted in the closed condition;
Figure 13 is a perspective view illustrating the third example of the tube holder fitted to the face of a user between the nose and the ear;
Figure 14 is a perspective view illustrating the third example of the tube holder being fitted with the tube;
Figure 15 is a perspective view illustrating the third example of the tube holder being moved to the closed condition;
Figure 16 is a perspective view illustrating a fourth example of the tube holder being fitted with the tube;
Figure 17 is a perspective view illustrating the fourth example of the tube holder in closed condition with the tube fitted in the tube holder;
Figure 18 is a perspective view illustrating the fourth example of the tube holder fitted to the face of a user between the nose and the ear;
Figure 19 is a perspective view illustrating the fourth example of the tube holder being fitted with the tube; and
Figure 20 is a perspective view illustrating the fourth example of the tube holder being fitted with the tube and being partially moved to the closed condition to cover the tube.

### Detailed Description

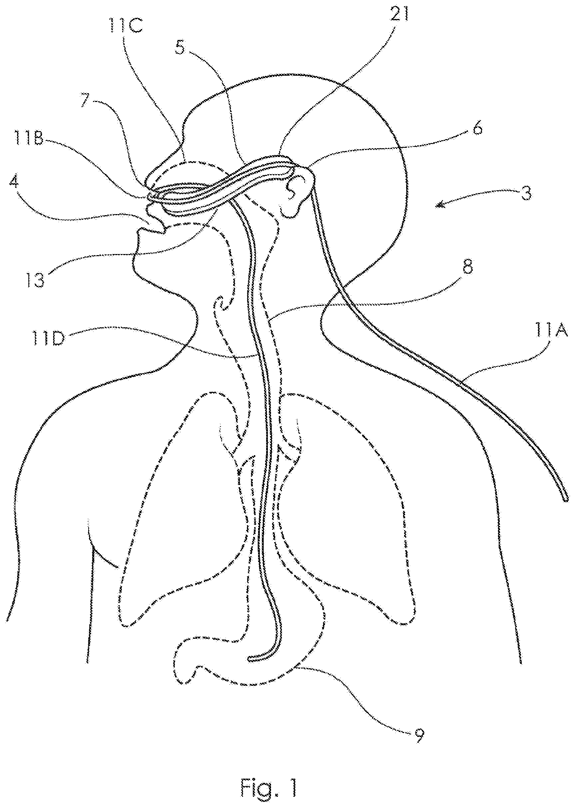

Referring to Figures 1 to 3, there is shown a first example of an apparatus 19 in the form of a tube holder 21 for mounting on a face 13 of the user 3 and holding and directing a tube 11 from an external source toward the nose or nostrils 7 of the user when tube 11 is mounted on the face 13. In particular, the tube holder 21 is arranged to extend across a cheek 5 of the face 13 to locate and direct the tube 11 from an ear 6 of the user 3 toward or into one of the nostrils 7 of the user 3.

In this example, the tube 11 may be a nasal gastric tube 11. However, other tubes or tube like structures may be utilized with the tube holder 11 such as oxygen tubes or even wires which need to be directed from an external source toward the user. It is also noted that whilst tube holder 21 is primarily described as directing the tube 11 toward or into the nostrils 7, the tube holder 21 may also be utilized to direct the tube 11 into a mouth 4 of the user 3.

Considering now the specific application of a nasal gastric tube 11 in more detail, the nasal gastric tube 11 is a continuous tube used for feeding and includes the feed section 11A from the food source feeding to an entry portion 11B at the nostrils 7 of the user, into the section 11C extending through the nasopharynx and section 11D extending through the esophagus 8 into the stomach 9.

In this particular application, there are a number of considerations. Firstly, the face of the user 3 has soft tissue over the cheek 5 and therefore attachment at that point is uncomfortable and particularly affected when tube holder 21 is attached and reattached. Secondly, the nasal gastric tube 11 needs to be directionally fed to the nostrils 7 and preferably from above the mouth 4 so as to not further inconvenience the user. Thirdly, the tube section 11C extending internally of the nostrils 7 and to the nasopharynx needs to be retained fairly stationary as constant movement will cause severe irritation and further could cause expelling of internal fluids out of the nose 7 or into the back of the mouth 4 and thereby further irritating the user. Fourthly, the tube section 11D needs to extend into the stomach 9 of the user and be retained at the right position so that food is directed to the right location and level in the stomach and not cause choking or blockage or incorrect processing or other complications if at the wrong position. Also it is important to not damage the inner lining of the stomach 9. Accordingly, it is important that the tube holder 21 secures the tube 11 so as to be substantially immobile and is attached or affixed to the face 13 in a manner so as to not irritate or damage the skin of the face 13.

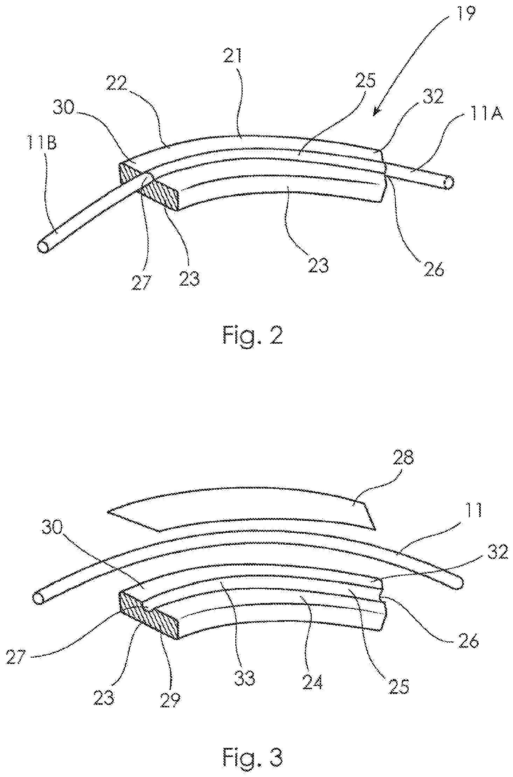

With the above considerations in mind, the first example of the tube holder 21 is now described in more detail with reference to Figures 2 to 5, the first example of tube holder 21 includes a body 22 for holding the nasal gastric tube 11 and an attachment means 23 such as an adhesive substance or gel for mounting or affixing the body 22 for holding the nasal gastric tube 11 on the user's face 3. The body 22 includes a first end 30 arrange in use toward or at the nostrils 7 and a second opposing ends 32 arranged at or toward the ear 6.

The body 22 of the nasal gastric tube holder 21 includes a directing means or locator 33 in the form of a channel 25 extending longitudinally along a central section of an upper surface 24 of the body 22 between a channel opening 26 at a one end 32 of the body and a channel outlet 27 at the other end 30 of the body 22.

Accordingly, the channel 25 assists to hold the nasal gastric tube 11 at a particular relative position to the body 22 to ensure correct length of the nasal gastric tube 11 extending from the body 22 to the nasal cavity 7 of the user 3 when in position and into the stomach 9.

The upper surface 24 of the body 22 may be a clear silicone material to provide a cleanable outer surface with the under surface preferably being waterproof and not skin irritant so as to be comfortably rested on the face of the user. An underside or backing surface 29 of the body 23 may include the attachment means 23 in the form of a layer of adhesive gel such as a hydrocolloid gel, which may be for example, Duoderm™.

The body 22 may be fitted with a cover 28 so as to form a sheath over the tube 11 when received by the channel 25. The cover 28 includes an adhesive underside to adhere to the upper surface 24 of the body 22 and the smooth water resistant or waterproof outer surface to protect the underlying body 22 and tube 11. In use, the cover 28 thereby serving to hold and shield the nasal gastric tube 11 in the nasal gastric tube holder 21. In this way, in position the channel 25 receives the nasal gastric tube 11 close to the ear 6 and directs the tube 11 to the nostril 7 of the user 3.

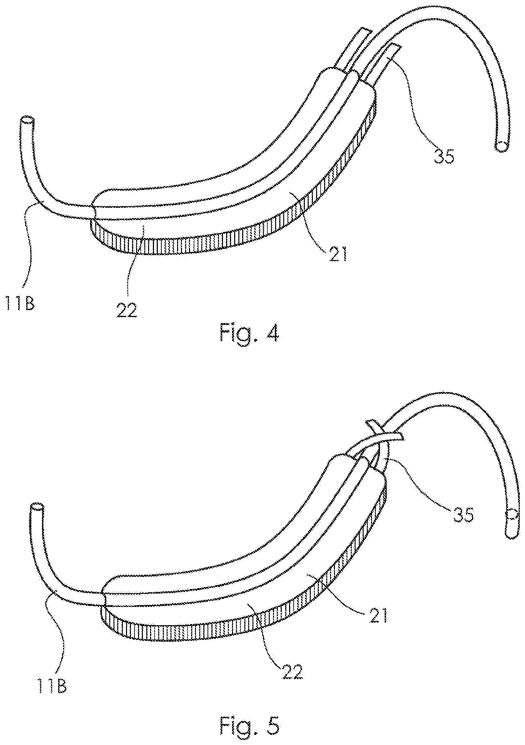

In other example forms, the channel 25 may remain uncovered but have means to retain the nasal gastric tube 11 in the channel 25 at least at the channel opening 26 at one end of the body 22 and at the channel outlet 27 at the other end of the body 22. An example of such a means is further described below with reference to Figures 4 and 5. The nasal gastric tube holder 21 can include various types of directing means or locators for directing the tube from an external source towards the nostrils 7 of the user when the nasal gastric tube 11 is mounted on the user's face 13. In particular the body 22 may have a first locating part (not shown) at the start 26 of the body 22 close to the ear 6 and have a second locating part (not shown) at the end 27 of the body 22 close to the nostrils 7.

Referring more specifically to Figures 4 and 5, in some forms, the first example of the tube holder 21 includes a holding or securing means provided in the form of tabs 35 at the start of the body 26 for tying around and holding the tube 11 at a longitudinally consistent position relative to the body 22 and when fixed to the face of the user 3 at a longitudinally consistent position to maintain the correct length of extent of the tube, 11B, 11C and 11D from the nasal gastric tub holder 21 when the nasal gastric tube is mounted on the user's face 13 and into the user's nostrils 7.

With the nasal gastric tube holder 21 attached to a particular relative position to the body a part of the extent of the nasal gastric tube 11A is fixed along the body 22 so that the portion 11B, 11C and 11D entering the nostrils 7 of the user 3 and through the nasopharynx to the esophagus 8 to the stomach 9 is fixed in length relative to the user to ensure correct positioning.

The body 22 is preferably flexible and mouldable to contour to the particular shape of user's face. The body 22 may be hand manipulated so as to readily allow shaping to a shape at which it remains that follows the contours of the face. In some example, the body 22 may include a thermosettable material to be moulded to a fixed form which is shaped to contour to the shape of user's face.

In use or operation the nasal gastric tube holder 21 is applied to face 13 similar to a Bandaid™ and holds the nasal gastric tube 11 substantially inside the gastric tube holder 21. The holder 21 is preferably generally waterproof, with a clear water proof cover 28 and may be moulded or shaped to fit around nose 7, the contour along the cheek 5 and up to the top of ear 6 adhering to face 13. The overall nasal gastric tube holder 21 is preferably lightweight.

The above described first example of the nasal gastric tube holder 21 may be utilised in a method of holding a nasal gastric tube 21 for a user 3 to be fed including the steps of providing a body 22 to hold and direct the nasal gastric tube 11, and attaching the body 22 to the user's face between the ear 6 and the nostrils 7 of the user 3. The method may also include the steps of shaping the body to fit with or contour in accordance with the contour or shape of the user's face 3.

Also the steps allow directing the nasal gastric tube at a particular relative position to the body to ensure directing the nasal gastric tube in a controlled manner by extending between a nasal gastric tube receiving position near the ear and exiting near the nostril of the user when in position and holding the nasal gastric tube relative to the body of the nasal gastric tube holder to a particular relative position to the body to ensure correct length of the nasal gastric tube extending from the body to the nasal cavity of the user when in position and locating the nasal gastric tube relative to the body.

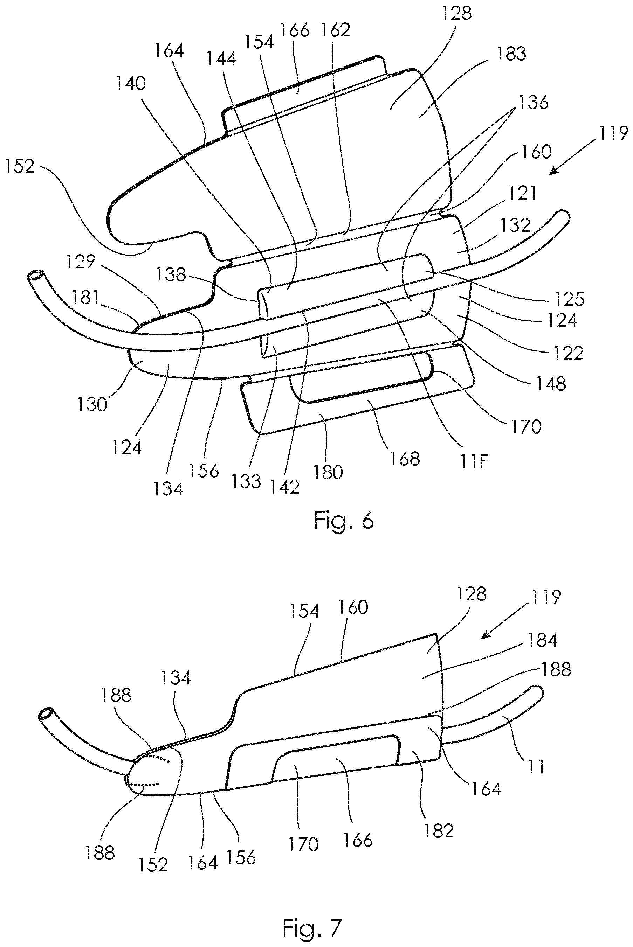

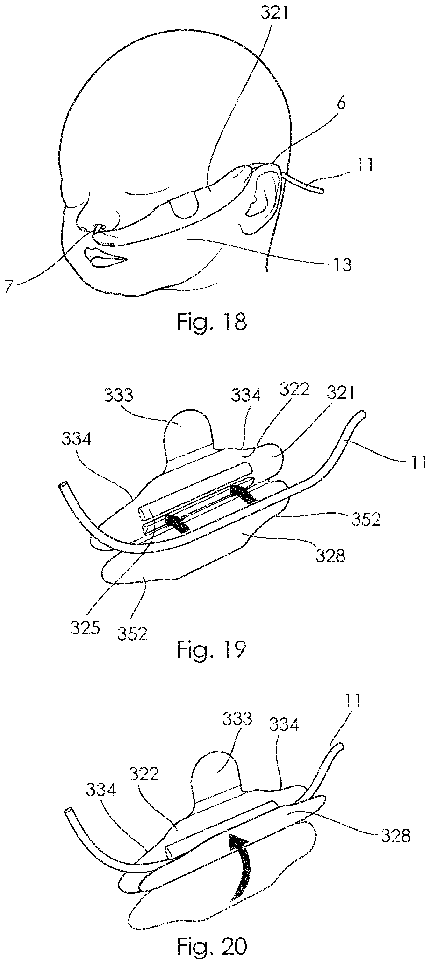

Referring now to Figures 6 to 20, there is provided further examples of the apparatus 19 in the form of the tube holder 21 in which like numerals denote like or similar parts. These examples are preferred examples of the invention and include further advantageous shaping, materials and functionality in comparison to the first example described above with reference to Figures 1 to 5.

Referring more specifically to Figures 6 to 10, there is provided a second example of an apparatus 119 in the form of a tube holder 121 in which like numerals or sequences of numerals denote like or similar parts. For example, body 22 in the first example relates to the body 122 in the second example.

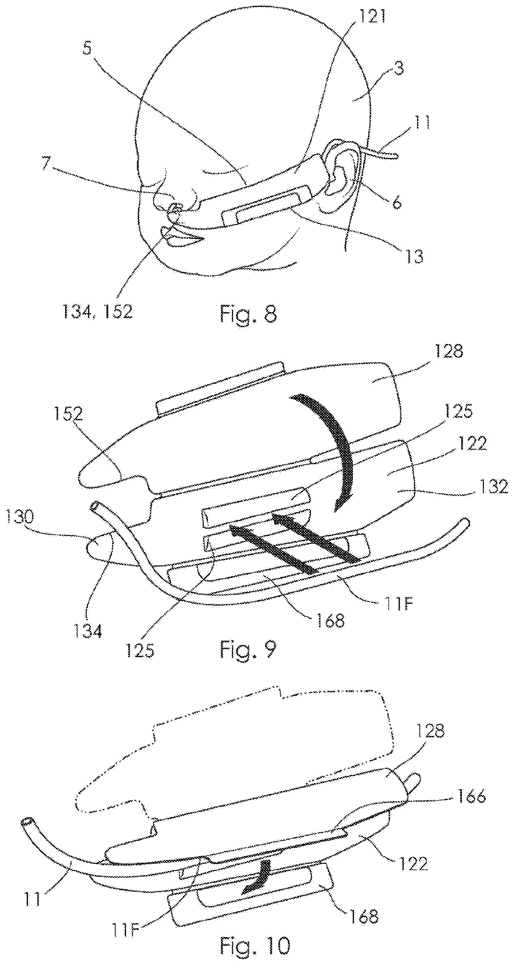

The tube holder 121 includes an elongate body 122 having a first end or nose end 130, a second end or opposing ear end 132, an underside or backing 129 adapted to be securable to the face of the user 3 and a locator 133 provided in the form of a channel 125 extending lengthwise at least partially between the nose end 130 and the ear end 312. The channel 125 is adapted to receive an elongate correspondingly received portion 11F of the nasal gastric tube 11 so as to maintain the orientation of the corresponding portion 11F substantially inline with the elongate body 122 and thereby between the ear 6 and nose 7 of the user 3 as is best shown in Figure 8.

The elongate body 122 includes a tapered or curved fitting portion 134 that tapers or concaves toward the nose end 130 so as to allow positioning of the tube holder 121 at least partially below and adjacent to the nostril 7 of the user 3, as is best shown in Figure 8. In this example, the channel 125 terminates short of the nose end 130 and is arranged so as to allow the nasal gastric tube 11 to pass across the nose-fitting portion 134 to the nostril 7 whilst the nose end 130 of the elongate body 122 continues to extend at least partially below the nostril 7. Accordingly, the elongate body 122 fits with the shape of the nostril 7 and allows the tube 11 to be fitted and secured immediately below the nostril 7.

In more detail, in this example, the channel 125 is provided by a channel arrangement 148 including two elongate and parallel foam parts 136 which define the channel 125 therebetween. The foam parts 136 have a flat underside 138 so as to fit with an upper side 124 of the body 122 and a curved upper side 140 which taper from an inner side 142 adjacent the channel 125 to the outer side 144 adjacent the body 122. The foam parts 136 are formed from a flexible foam material such as polyurethane foam so as to bend or contour to the shape of the face 13 of the user 3. The height of the channel arrangement 148, more specifically the foam parts 136, which defines the depth or height of the channel 125, is about the same or slightly greater than the diameter of the tube 11 which depending on the application may be about 3mm to 6mm. The width of the channel 125, defined by the distance between the foam parts 136, may be about the same or slightly less than the diameter of the tube 11 so as to locate and snuggly received the tube 11 in the fitted condition.

Accordingly, the channel 125 serves to locate and retain the tube 11 between the first and second ends 130, 132 and hold the tube 11 in position whilst the position of the tube 11 is assessed. For example, the method of fitting a nasal gastric tube 11 includes the step of drawing back on the tube 11 when initially fitted to check that the tube 11 is within the stomach 9. Then once the tube 11 is determined to be in the correct location, a cover 128, as is further described below, is fitted over the tube 11 within the channel 125 and the elongate body 122 to secure the tube 11 in place.

In this example, the tube holder 121 further includes a folding cover or flap 128 pivotally attached or coupled to the elongate body 122. The cover 128 is moveable between an open condition, as shown in Figure 6, in which the tube 11 is fitted and accessible and a closed condition, as shown in Figure 7, in which the tube 11 is at least partially concealed by the cover 128. The cover 128 is attached at seam or fold line 160 and is substantially symmetrical in shape with the elongate body 122 about the fold line 160. Accordingly, the cover 128 is shaped to substantially match the shape of the elongate body 122 when in the closed condition and has a corresponding fitting portion 152 arranged to align with the fitting portion 134 of the elongate body 122 in the closed condition. Accordingly, the cover 128, which is preferably made from a splash or waterproof material, serves to cover and seal with elongate body 122 and the tube 11 such that the tube holder 121 is substantially waterproof.

The elongate body 122 includes a first side 154 and second opposing side 156 extending between the nose end 130 and the ear end 132. The cover 128 includes an attached side 162 pivotally coupled via the fold line 160 to the first side 154 of the elongate body 122 and an opposing free end side 164 which substantially meets with the second side 156 of the elongate body 122 in the closed condition. The cover 128 includes a first tab 166 coupled to free end side 164 and the elongate body 122 includes second tab 168 coupled to the second side 156 of the elongate body 122. The second tab 168 includes an aperture 170 arranged to receive the first tab 166 to secure the cover 128 in the closed condition.

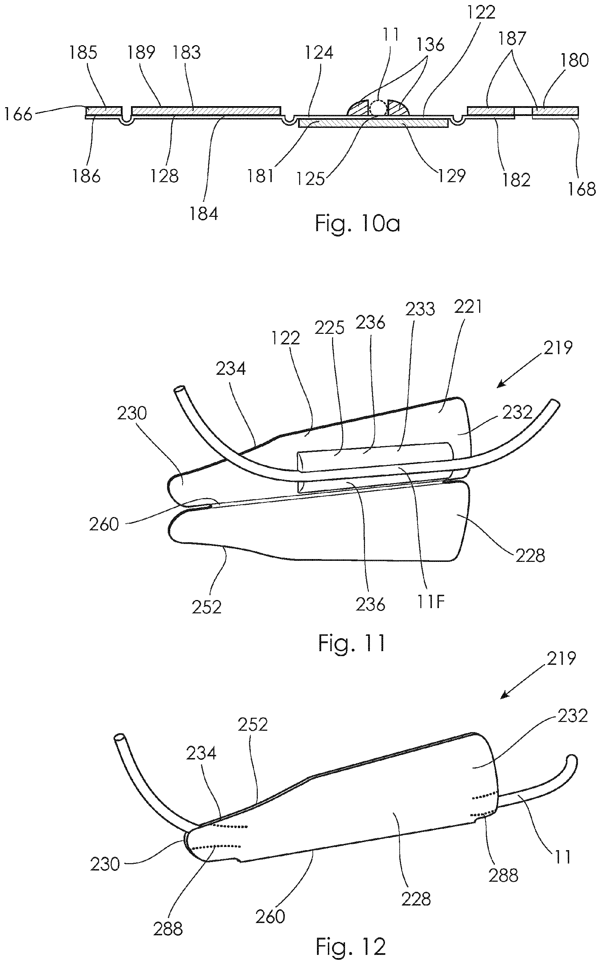

Turning now to the materials of construction, and referring additionally to Figure 10a, in this example, the body 122 may be substantially formed from a flexible polyurethane or urethane material. The underside 129 of the body 122 may include or carry an adhesive layer 181 arranged to be adhered or fixed to the face 13 of the user and the topside or upper side 124 of the body 122 may be substantially free of adhesive to allow for movement, such as sliding, and positioning of the tube 11 within and relative to the channel 125. An inner face 180 of second tab 168 of the body 122 may include or carry an adhesive layer 187 such as an acrylic adhesive which is arranged to fix to an outer surface 184 of the cover 128 in the closed condition. The outer face 182 of the second tab 168 is substantially free of adhesive.

In some forms, the adhesive layer 181 may include a substance such as an adhesive gel such as a hydrocolloid substance or gel, which may be similar to, for example, Duoderm™. The hydrocolloid gel may be carried by the upper layer 124 which may be thin flexible polyurethane or urethane material. The foam parts 136 may then be adhered to the opposing side of upper layer 124 relative to the hydrocolloid gel.

The cover 128 is also preferably made from a flexible polyurethane or urethane material and may be formed from the same piece of material as the body 122 or at least the same material as the upper surface or layer 124 of the body 122. In some forms, the cover is a urethane breathable non-woven sheet having an acrylic adhesive such as the ST-284 urethane sheet available from Nitto™. The cover 128 is waterproof.

In some applications, the cover 128 is clear, opaque or may include a clear window to allow for visual inspection of any liquids passing through or within the tube 11. The cover 128 includes an outer side 184 and an opposing inner side 183 which carries an adhesive substance or layer 189 to adhere or fix to the body 122 in the closed condition. The first tab 166 may also include an adhesive layer 185. As is best shown in Figure 7, the cover 128 may include pre-defined perforations 188 to accommodate the tube 11. The pre-defined perforations 188 allow a user fitting the tube to pinch-fit the cover 128 to the tube 11 to assist with sealing the cover 128 around the tube 11 which assists with the overall water resistance or water proof structure of the tube holder 121. In other examples, the cover 128 may be a woven material or a skin coloured material.

In this example, the overall length of the tube holder 121 is about 8 cm to 10 cm so as to span between the nose 7 and ear 6 of an infant or child. However, other sizes may be utilised. For example, an adult version may be shorter relative to the face of the adult and only extend part of the way from the nose toward the ear of the adult use. The materials of the tube holder 121 including the flexible elongate body 121 and flexible cover 128 allow the tube holder 121 to fit with and contour with a variety of face shapes and sizes. It is noted that in this example the tube holder 121 is shaped for use on the left-hand side of the face 13. However, tube holder 121 may also be formed in a right-hand version for fitting with the right-hand side of the face 13.

Referring to Figure 9 and 10, in use, the tube holder 121 may be provided in a sealed package (not shown). The package is opened and a protective film or paper is removed from the underside or backing 129 of the body 122 to reveal the adhesive surface 181 which allows the body 122 to be adhered or fixed to the face 13 of the user 3 between the nose 7 and the ear 6 as is shown in Figure 8. The tube 11, more specifically the received portion 11F of the tube 11, is then fitted to the channel 125. The upper surfaces of the body 122 may be free of adhesive to allow the positioning or sliding of the tube 11 relative to the channel 125. The tube 11 may carry a marking to ensure the correct length of tube 11 proceeds into the stomach as is best shown in Figure 1. The inner face 183 of the cover 128 may also initially be covered by a protective film or paper which is removed to reveal the adhesive carried by the surface 183 prior to the cover 128 being moved to the closed condition.

Referring now to Figure 11 to 15, there is shown a third example of an apparatus 219 in the form of a tube holder 221 for securing the tube 11 to the face 13 of the user 3. This example is similar to the second example as described above with reference to Figure 6 to 10. Accordingly, all parts are not described here again and like numerals are used to denote like or similar parts.

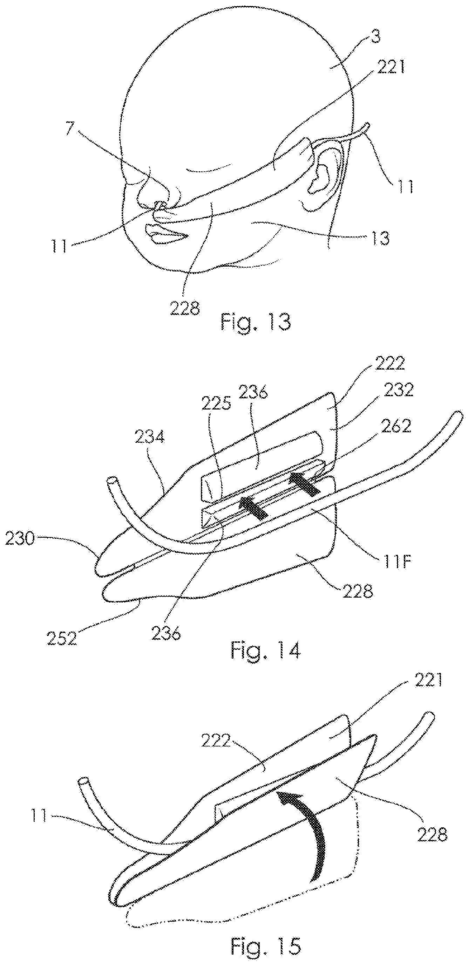

In this third example, the tube holder 221 has been simplified by removal of the first and second tabs 166, 168. Accordingly, the body 222 and the cover 228 substantially mirror one another in a butterfly type arrangement being symmetrical about the fold joint 260. In this example, the fold joint is provided on the lower side of the tube holder 221 in the closed and fitted condition. However, the fold joint 260 could also be provided on the upper side of the tube holder 221 in the closed and fitted condition, similar to that shown in Figures 6 to 10. The body 222 and the cover 228 are each correspondingly shaped to provide the concave fitting portions 234, 252 which unite in the closed condition so as fit beneath the nostrils of the user 3 and allows the tube 11 to pass across the tube holder 221 and upwardly into the nostrils 7 as is best shown in Figure 13. The materials of construction and method of use are similar to those described in relation to second example above with reference to Figures 6 to 10 and are not described again here.

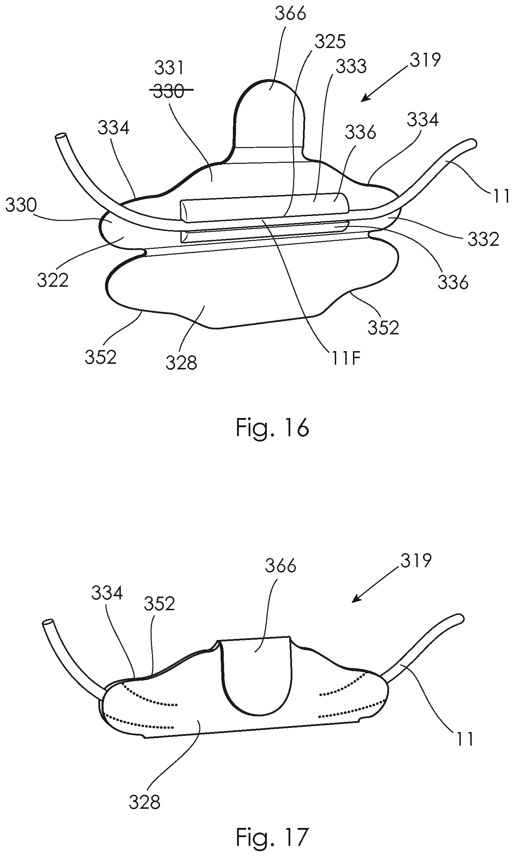

Referring now to Figures 16 to 20, there is shown a fourth example of the tube holder 321 for securing the tube 11 to the face 13 of the user 3. This example is similar to the second example as described above with reference to Figure 6 to 10. Accordingly, all parts are not described here again and like numerals are used to denote like or similar parts.

In this example, the tube holder 321 is symmetrical so as to be fitted with the nose or nostril 7 of the user 3 along either the right hand side or left hand side of the face 13. In more detail, in this fourth example, the elongate body 322 tapers toward both of the first end 330 and second end 332 so as to include symmetrical nose receiving or fitting portions 334 toward each of the first and second ends 330, 332. In this example, the elongate body 322 having a wider central section 331 that carries a securing tab 366 that is folded over the cover 328 when the cover 328 is in the closed condition. Similar to the previous example, the cover 328 has a similar shape to the elongate body 322 so as to fit with and match the shape of the elongate body 322 when the cover 328 is in the closed condition.

Similar to the second and third examples of the tube holder 21 , in this example, the locator 333 is provided in the form of the channel 325 which extends between the first end 330 and second end 332 of the elongate body 322. The channel 325 is centered between the first end 330 and second end 332 and terminates short of the first end 330 and second end 332 such that the tube 11 is free to extend from the channel 325 and across the respective nose-fitting portions 334 in use. This allows the received portion of the tube 11 to be held and aligned by the channel 325 whilst the tube 11 is being positioned within the nostril 7 of the user 3. The cover 328 which includes the inner adhesive securing surface 383 is then closed over the fitted tube 11 and the ends of the tube 11 extending from the receive portion 11F are directly captured and fixed in the desired orientation relative to the nostril 7 of the user 3. The securing tab 366 secures and seals the cover 328 in the closed condition.

In each of the second, third and fourth examples, tube holders 121, 221, 321 broadly function in a similar way. The example below is described with reference to the second example of the tube holder 121 but is equally applicable to the other examples.

Accordingly, a method of retaining a tube 11 using the tube holder 121 on the face 13 of the user 3 between the nose 7 of the user 3 and the ear 6 of the user 3 may include the methods steps of: affixing the securing side 129 of an elongate body 122 of the tube holder 121 such that the elongate body 122 is substantially aligned between the nose 7 and the ear 6. The method then includes locating a received portion 11F of the tube 11 using the locator 133 of the elongate body 122 such that the received portion 11F of the tube 11 is substantially inline with the elongate body 122. The received portion 11F may include a marking to ensure the tube 11 is of the correct length.

The method may also include the steps of positioning of the tube holder 121 with a nose fitting portion 134 at least partially below and adjacent to the nostril 7 of the user 3 and fitting the tube 11 to the locator 133 so as to extend from the locator 133 across the nose fitting portion 134 and into the nostril 7 of the user 3.

Advantageously, having the locator 133 in the form of the channel 125 functions to maintain overall lengthwise alignment of the tube 11 and provides a structure to hold tube and allow for moveable adjustment of the tube 11 before the cover 128 is moved to the closed condition. The ends of the tube 11 which extend from the channel 125 and across the nose-fitting portion 134 are typically moved and adjusted for each user 3 to achieve a customised fit with the nostril 7.

Accordingly, once the location of the tube 11 has been determined to be correct, the method further includes the step of: moving the cover 128 coupled to the elongate body 122 from an open condition to a closed condition to cover the tube 11 when the tube 11 is received by the locator 133. The cover 128 being arranged to fit with and have a substantially similar shape to the body 122. The cover 128 is affixed to or secures against the elongate body 122 to retain and secure the tube 11 in the desired location. In particular, because the channel 125 terminates short of the first end 130 of the body 122, the portion of the tube 11 between the nostril 7 and the channel 25 may be bent and shaped for a customised fit. The cover 128 is then directly secured around the tube 11 to fix the tube 11 in the customised position and within the channel 25. Furthermore, the concave or tapered end of the body 122 which provides the nose fitting portions 134, 152 allows the body 12 and adhesive cover 128 to be placed very near and beneath the nostril 7 which further assist to secure the position and location of the tube 11 relative to the nostril 7. The remainder of the elongate body 122 and, in particular the channel 125, then serving to securely support and direct the tube 11 along the cheek 5 and preferably to a position over the ear 6. The cover 128 being arranged to substantially cover the tube 11 between the nostril 7 and the ear 6 which advantageously assists to inhibit a user, such as child or toddler, from clasping the tube 11 and attempting to dislodge the tube 11.

The above described apparatus and method provide a number of advantageous features and functionality. In particular, the tube holder allows for a single caregiver to fit the tube to the user as the locator provides for temporary support of the tube whilst the overall length and positioning of the tube is checked. The pre-shaped attached cover can then be simply folded over the body and tube to secure the tube. The shaping of the elongate body and cover is also highly advantageous to allow very close positioning to the nostrils and the channel terminating short of the ends of the body allow the portion of tube between the channel and nostril to be custom fitted and secured in the custom position directly by the cover. The cover, when fitted, serves to protect the tube holder and when securing tabs are used, the securing tabs provide a secondary measure to ensure that the cover remains closed. The tube holder has a unitary and substantially waterproof structure which assists it to remain attached to the face for longer periods of time.

Other advantageous aspects of the above described apparatus and method include: quicker and easier to apply and fit an oral or a nasal gastric tube. Easier to insert by fitting the tube to the locator at the correct stomach measurement; Longer lasting in position so as to minimize times for replacement or reattachment of nasal gastric tube; nasal gastric tube holder will last longer- contouring shaped design to mould to the face, waterproof adhesive similar to Duoderm™ with clear silicone or skin tone top; Less traumatic for the patient (no need to change frayed tape 2 to 3 times per week); User friendly - less stressful for the person such as parents or patient carer inserting the nasal gastric tube; Less traumatic for nasal gastric tube fed children and the parents, nurses and doctors inserting the tube; Correct measurement pre- marked of tube relative to tube holder by inserting the nasal gastric tube at the predefined location in the nasal gastric tube holder; More hygienic; Looks better than messy tape and cleaner design with smooth surface, which is less noticeable on the skin. Nasal gastric tube holder is one shaped design corresponding with patient's face; nasal gastric tube holder feeds the nasal gastric tube close to the nostril and therefore directs well and minimizes irritation and likely removal by patient; Children may not become as aversive to the nasal gastric tube with the nasal gastric tube holder due to it being quicker and lasting longer.

The nasal gastric tube holder can be provided in a pack of six (3 x left face nasal gastric tube holders and 3 x right face nasal gastric tube holders). The tube holders may be hygienically wrapped until use to maintain sterility.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any known matter or any prior publication is not, and should not be taken to be, an acknowledgment or admission or suggestion that the known matter or prior art publication forms part of the common general knowledge in the field to which this specification relates.

While specific examples of the invention have been described, it will be understood that the invention extends to alternative combinations of the features disclosed or evident from the disclosure provided herein.

Many and various modifications will be apparent to those skilled in the art without departing from the scope of the invention disclosed or evident from the disclosure provided herein.

## Claims

1. An apparatus (19) for securing a nasal gastric tube (11) to a face (13) of a user (3) between an ear (6) of the user and a nose (7) of the user in a fitted condition, the apparatus comprising:
an elongate body (122) having a securing side (129) adapted to affix to the face of the user; and
a tube locating side (124) including a locator (133) arranged to receive and locate a received portion (11F) of the tube substantially inline with the elongate body between the ear and nose of the user;
a cover (128) coupled to the elongate body so as to be moveable between an open condition in which the received portion of the tube is receivable by the locator and a closed condition in which the received portion of the tube is concealed by the cover;
wherein the elongate body includes a first end (130) oriented toward the nose in the fitted condition and an opposing second end (132) oriented toward the ear in the fitted condition, and wherein the elongate body is arranged to narrow toward the first end so as to provide a nose fitting portion (134) shaped to at least partially complement the shape of the nose proximate a nostril (7) thereof so as to allow positioning of the elongate body at least partially below and adjacent to the nostril.

2. The apparatus according to claim 1, wherein the locator is arranged to temporarily movably hold the received portion of the tube in the open condition so as to allow for moveable adjustment of the tube prior to the cover being moved to the closed condition.

3. The apparatus according to claim 1 or claim 2, wherein the elongate body is dimensioned to extend at least half way between the nose and the ear of the user.

4. The apparatus according to any one of claims 1 to 3, wherein the elongate body is arranged to narrow toward the second end so as to provide a second nose fitting portion so as to allow the tube holder to be fitted to the face in a right-hand or a left-hand orientation.

5. The apparatus according to any one of claims 1 to 4, wherein the cover is shaped to substantially match the shape of the elongate body and has a corresponding nose fitting portion arranged to align with the nose fitting portion of the elongate body in the closed condition, and, optionally, wherein the locator is provided in the form of a channel shaped to receive and locate the received portion of the tube, the channel extending lengthwise at least partially between the first end and the second end of the elongate body.

6. The apparatus according to claim 5, wherein the channel terminates short of the first end and is arranged so as to allow the nasal gastric tube to pass across the nose fitting portion to the nostril whilst the first end of the elongate body continues to extend at least partially below the nostril.

7. The apparatus according to any one of claims 1 to 3, wherein elongate body includes first and second opposing sides extending between the first and second ends, and wherein the cover includes an attached side coupled to the first side of the elongate body and an opposing free end side which substantially meets with the second side of the elongate body in the closed condition, and, optionally, wherein the second side of the elongate body includes a securing tab which is arranged to fold at least partially over the cover in the closed condition thereby securing the cover in the closed condition.

8. The apparatus according to claim 5, wherein the channel is defined by two elongate members coupled to the locating face of the elongate body, the two elongate members being arranged in parallel and spaced apart from one another.

9. The apparatus according to claim 8, wherein the base of the channel is provided by the locating side of the elongate body such that in the fitted condition the tube lies substantially along the locating side, and/or wherein the elongate members are formed from a flexible foam material.

10. The apparatus according to any one of claims 1 to 9, wherein the cover is at least one of clear or translucent so as to allow visual inspection of the tube beneath the cover.

11. The apparatus according to any one of claims 5 to 9, wherein the cover includes an inner securing surface and an outer surface, the inner securing surface being adapted to affix to the locating side of the elongate body in the closed condition and the outer surface being substantially free of adhesive, and, optionally, wherein the cover and elongate body are formed from a unitary material being at least one of a urethane or polyurethane and/or the locating side includes at least one of a silicone, urethane or polyurethane material.

12. The apparatus according to any one of claims 1 to 11, wherein the securing side of the elongate body includes a hydrocolloid substance adapted to adhere to the skin.

13. The apparatus according to any one of claims 1 to 12,wherein the elongate body includes a first end (130) oriented toward the nose in the fitted condition and an opposing second end (132) oriented toward the ear in the fitted condition, and wherein the elongate body is arranged to narrow toward the first end so as to provide a nose fitting portion (134) shaped to at least partially complement the shape of the nose proximate a nostril (7) thereof so as to allow positioning of the elongate body at least partially below and laterally adjacent to the nostril.

14. A method of retaining a tube (11) on a face (13) of a user (3) between a nose (7) of the user and an ear (6) of the user, the method including the steps of:
a. affixing a securing side (129) of an elongate body (122) of a tube holder (121) to the face such that the elongate, body is substantially aligned between the nose and the ear;
b. locating a received portion (11F) of the tube using a locator (133) coupled to the elongate body and adapted to receive the received portion such that the received portion of the tube is substantially inline with the elongate body;
c. moving a cover (128) coupled to the elongate body from an open condition to a closed condition to cover the tube when the tube is received by the locator;
d. positioning a nose fitting portion (134) of the tube holder at least partially below and laterally adjacent to a nostril (7) of the nose of the user, the nose fitting portion shaped to at least partially complement the shape of the nose proximate the nostril thereof; and
e. fitting the tube to the locator so as to extend from the locator across the nose fitting portion and into the nostril of the nose of the user.

## Patentansprüche

1. Vorrichtung (19) zum Befestigen einer nasalen Magensonde (11) an einem Gesicht (13) eines Benutzers (3) zwischen einem Ohr (6) des Benutzers und einer Nase (7) des Benutzers in einem montierten Zustand, wobei die Vorrichtung Folgendes umfasst:
einen länglichen Körper (122) mit einer Befestigungsseite (129), ausgelegt zum Fixieren am Gesicht des Benutzers; und
eine Sondenpositionierungsseite (124) mit einer Positionierhilfe (133), ausgelegt zum Aufnehmen und Positionieren eines aufgenommenen Abschnitts (11F) der Sonde im Wesentlichen inline mit dem länglichen Körper zwischen dem Ohr und der Nase des Benutzers;
eine Abdeckung (128), die so mit dem länglichen Körper gekoppelt ist, dass sie zwischen einem offenen Zustand, in dem der aufgenommene Abschnitt der Sonde in der Positionierhilfe aufgenommen werden kann, und einem geschlossenen Zustand beweglich ist, in dem der aufgenommene Abschnitt der Sonde von der Abdeckung verborgen wird;
wobei der längliche Körper ein erstes Ende (130), das in Richtung der Nase im montierten Zustand orientiert ist, und ein gegenüberliegendes zweites Ende (132) aufweist, das in Richtung des Ohrs im montierten Zustand orientiert ist, und wobei der längliche Körper so ausgelegt ist, dass er in Richtung des ersten Endes schmäler wird, um einen Nasenmontageabschnitt (134) zu bilden, der so gestaltet ist, dass er die Form der Nase in der Nähe eines Nasenlochs (7) davon wenigstens teilweise komplementiert, um eine Platzierung des länglichen Körpers wenigstens teilweise unter und neben dem Nasenloch zuzulassen.

2. Vorrichtung nach Anspruch 1, wobei die Positionierhilfe zum vorübergehenden beweglichen Halten des aufgenommenen Abschnitts der Sonde im offenen Zustand ausgelegt ist, um eine bewegliche Justierung der Sonde vor dem Bewegen der Abdeckung in den geschlossenen Zustand zuzulassen.

3. Vorrichtung nach Anspruch 1 oder Anspruch 2, wobei der längliche Körper so dimensioniert ist, dass er wenigstens in der Hälfte zwischen Nase und Ohr des Benutzers verläuft.

4. Vorrichtung nach einem der Ansprüche 1 bis 3, wobei der längliche Körper so ausgelegt ist, dass er in Richtung des zweiten Endes schmäler wird, um einen zweiten Nasenmontageabschnitt bereitzustellen, so dass der Sondenhalter am Gesicht in einer rechten oder linken Orientierung montiert werden kann.

5. Vorrichtung nach einem der Ansprüche 1 bis 4, wobei die Abdeckung so gestaltet ist, dass sie im Wesentlichen zur Gestalt des länglichen Körpers passt und einen entsprechenden Nasenmontageabschnitt aufweist, ausgelegt zum Ausrichten mit dem Nasenmontageabschnitt des länglichen Körpers im geschlossenen Zustand, und wobei die Positionierhilfe optional in Form eines Kanals ausgebildet ist, der zum Aufnehmen und Positionieren des aufgenommenen Abschnitts der Sonde gestaltet ist, wobei der Kanal längsweise wenigstens teilweise zwischen dem ersten Ende und dem zweiten Ende des länglichen Körpers verläuft.

6. Vorrichtung nach Anspruch 5, wobei der Kanal kurz vor dem ersten Ende endet und so ausgelegt ist, dass er es zulässt, dass die nasale Magensonde über den Nasenmontageabschnitt zum Nasenloch passiert, während das erste Ende des länglichen Körpers weiter wenigstens teilweise unter dem Nasenloch verläuft.

7. Vorrichtung nach einem der Ansprüche 1 bis 3, wobei der längliche Körper eine erste und eine gegenüberliegende zweite Seite aufweist, die zwischen dem ersten und zweiten Ende verlaufen, und wobei die Abdeckung eine angebrachte Seite, die mit der ersten Seite des länglichen Körpers gekoppelt ist, und eine gegenüberliegende freie Endseite aufweist, die im Wesentlichen mit der zweiten Seite des länglichen Körpers im geschlossenen Zustand zusammentrifft, und wobei optional die zweite Seite des länglichen Körpers eine Verschlusslasche aufweist, die zum Falten wenigstens teilweise über die Abdeckung im geschlossenen Zustand ausgelegt ist, um dadurch die Abdeckung im geschlossenen Zustand zu befestigen.

8. Vorrichtung nach Anspruch 5, wobei der Kanal von zwei länglichen Elementen definiert wird, die mit der Positionierhilfefläche des länglichen Körpers gekoppelt sind, wobei die beiden länglichen Elemente parallel und voneinander beabstandet angeordnet sind.

9. Vorrichtung nach Anspruch 8, wobei die Basis des Kanals von der Positionierungsseite des länglichen Körpers gebildet wird, so dass die Sonde im montierten Zustand im Wesentlichen entlang der Positionierungsseite liegt, und/oder wobei die länglichen Elemente aus einem flexiblen Schaumstoff gebildet sind.

10. Vorrichtung nach einem der Ansprüche 1 bis 9, wobei die Abdeckung klar und/oder lichtdurchlässig ist, um eine visuelle Prüfung der Sonde unterhalb der Abdeckung zuzulassen.

11. Vorrichtung nach einem der Ansprüche 5 bis 9, wobei die Abdeckung eine innere Befestigungsfläche und eine äußere Fläche aufweist, wobei die innere Befestigungsfläche zum Fixieren an der Positionierungsseite des länglichen Körpers im geschlossenen Zustand ausgelegt ist und die äußere Fläche im Wesentlichen frei von Klebstoff ist, und wobei optional die Abdeckung und der längliche Körper aus einem einheitlichen Material gebildet sind, das Urethan und/oder Polyurethan ist, und/oder die Positionierungsseite wenigstens eines aus einem Silikon-, Urethan- oder Polyurethanmaterial beinhaltet.

12. Vorrichtung nach einem der Ansprüche 1 bis 11, wobei die Befestigungsseite des länglichen Körpers eine Hydrokolloidsubstanz aufweist, die zum Anhaften an der Haut ausgelegt ist.

13. Vorrichtung nach einem der Ansprüche 1 bis 12, wobei der längliche Körper ein erstes Ende (130) aufweist, das in Richtung der Nase im montierten Zustand orientiert ist, und ein gegenüberliegendes zweites Ende (132), das in Richtung des Ohrs im montierten Zustand orientiert ist, und wobei der längliche Körper so ausgelegt ist, dass er in Richtung des ersten Endes schmäler wird, um einen Nasenmontageabschnitt (134) zu bilden, der so gestaltet ist, dass er die Gestalt der Nase in der Nähe eines Nasenlochs (7) davon wenigstens teilweise komplementiert, um eine Platzierung des länglichen Körpers wenigstens teilweise unter und lateral neben dem Nasenloch zuzulassen.

14. Verfahren zum Halten einer Sonde (11) an einem Gesicht (13) eines Benutzers (3) zwischen einer Nase (7) des Benutzers und einem Ohr (6) des Benutzers, wobei das Verfahren die folgenden Schritte beinhaltet:
a. Fixieren einer Befestigungsseite (129) eines länglichen Körpers (122) eines Sondenhalters (121) am Gesicht, so dass der längliche Körper im Wesentlichen zwischen der Nase und dem Ohr ausgerichtet ist;
b. Positionieren eines aufgenommenen Abschnitts (11F) der Sonde mit einer Positionierhilfe (133), die mit dem länglichen Körper gekoppelt ist, und ausgelegt zum Aufnehmen des aufgenommenen Abschnitts, so dass der aufgenommene Abschnitt der Sonde im Wesentlichen inline mit dem länglichen Körper ist;
c. Bewegen einer mit dem länglichen Körper gekoppelten Abdeckung (128) von einem offenen Zustand in einen geschlossenen Zustand, um die Sonde zu bedecken, wenn die Sonde in der Positionierhilfe aufgenommen ist;
d. Platzieren eines Nasenmontageabschnitts (134) des Sondenhalters wenigstens teilweise unter und lateral neben einem Nasenloch (7) der Nase des Benutzers, wobei der Nasenmontageabschnitt so gestaltet ist, dass er die Gestalt der Nase in der Nähe des Nasenlochs davon wenigstens teilweise komplementiert; und
e. Montieren der Sonde an der Positionierhilfe, so dass sie von der Positionierhilfe über den Nasenmontageabschnitt und in das Nasenloch der Nase des Benutzers verläuft.

## Revendications

1. Appareil (19) de fixation d'une sonde naso-gastrique (11) sur le visage (13) d'un utilisateur (3) entre une oreille (6) de l'utilisateur et le nez (7) de l'utilisateur dans un état monté, l'appareil comprenant :
un corps allongé (122) ayant un côté de fixation (129) adapté pour être apposé sur le visage de l'utilisateur ; et
un côté de placement de sonde (124) comportant un élément de placement (133) conçu pour recevoir et placer une partie reçue (11F) de la sonde sensiblement dans le sens du corps allongé entre l'oreille et le nez de l'utilisateur ;
un couvercle (128) couplé au corps allongé de façon à être mobile entre un état ouvert dans lequel la partie reçue de la sonde peut être reçue par l'élément de placement et un état fermé dans lequel la partie reçue de la sonde est cachée par le couvercle ;
dans lequel le corps allongé comporte une première extrémité (130) orientée vers le nez dans l'état monté et une deuxième extrémité (132) opposée orientée vers l'oreille dans l'état monté, et dans lequel le corps allongé est conçu pour se rétrécir vers la première extrémité afin de créer une partie de montage sur le nez (134) conformée de manière à être au moins partiellement complémentaire de la forme du nez à proximité d'une narine (7) de celui-ci afin de permettre le positionnement du corps allongé au moins partiellement en dessous et adjacent à la narine.

2. Appareil selon la revendication 1, dans lequel l'élément de placement est conçu pour maintenir temporairement de manière mobile la partie reçue de la sonde dans l'état ouvert afin de permettre un ajustement mobile de la sonde avant que le couvercle soit déplacé à l'état fermé.

3. Appareil selon la revendication 1 ou la revendication 2, dans lequel le corps allongé est dimensionné pour s'étendre au moins à mi-distance entre le nez et l'oreille de l'utilisateur.

4. Appareil selon l'une quelconque des revendications 1 à 3, dans lequel le corps allongé est conçu pour se rétrécir vers la deuxième extrémité afin de créer une deuxième partie de montage sur le nez pour permettre le montage du support de sonde sur le visage dans une orientation à droite ou à gauche.

5. Appareil selon l'une quelconque des revendications 1 à 4, dans lequel le couvercle est conformé pour correspondre sensiblement à la forme du corps allongé et possède une partie de montage sur le nez correspondante conçue pour être alignée avec la partie de montage sur le nez du corps allongé dans l'état fermé, et, facultativement, dans lequel l'élément de placement est réalisé sous la forme d'un canal conformé pour recevoir et placer la partie reçue de la sonde, le canal s'étendant dans le sens de la longueur au moins partiellement entre la première extrémité et la deuxième extrémité du corps allongé.

6. Appareil selon la revendication 5, dans lequel le canal se termine en deçà de la première extrémité et est conçu pour permettre à la sonde naso-gastrique de traverser la partie de montage sur le nez vers la narine tandis que la première extrémité du corps allongé continue à s'étendre au moins partiellement en dessous de la narine.

7. Appareil selon l'une quelconque des revendications 1 à 3, dans lequel le corps allongé comporte des premier et deuxième côtés opposés qui s'étendent entre les première et deuxième extrémités, et dans lequel le couvercle comporte un côté rattaché couplé au premier côté du corps allongé et un côté d'extrémité libre opposé qui rejoint sensiblement le deuxième côté du corps allongé dans l'état fermé, et, facultativement, dans lequel le deuxième côté du corps allongé comporte une languette de fixation qui est conçue pour se replier au moins partiellement par dessus le couvercle dans l'état fermé afin de sécuriser ainsi le couvercle dans l'état fermé.

8. Appareil selon la revendication 5, dans lequel le canal est défini par deux éléments allongés couplés à la face de placement du corps allongé, les deux éléments allongés étant disposés en parallèle et espacés l'un de l'autre.

9. Appareil selon la revendication 8, dans lequel la base du canal est réalisée par le côté de placement du corps allongé de façon que, dans l'état monté, la sonde se trouve sensiblement le long du côté de placement, et/ou dans lequel les éléments allongés sont formés à partir d'un matériau en mousse flexible.

10. Appareil selon l'une quelconque des revendications 1 à 9, dans lequel le couvercle est l'un au moins de transparent ou translucide afin de permettre une inspection visuelle de la sonde sous le couvercle.

11. Appareil selon l'une quelconque des revendications 5 à 9, dans lequel le couvercle comporte une surface de fixation intérieure et une surface extérieure, la surface de fixation intérieure étant adaptée pour être apposée sur le côté de placement du corps allongé dans l'état fermé et la surface extérieure étant sensiblement dépourvue d'adhésif, et, facultativement, dans lequel le couvercle et le corps allongé sont formés à partir d'un matériau unitaire qui est l'un au moins parmi un uréthane ou un polyuréthane, et/ou le côté de placement comporte l'un au moins parmi un matériau silicone, uréthane ou polyuréthane.

12. Appareil selon l'une quelconque des revendications 1 à 11, dans lequel le côté de fixation du corps allongé comporte une substance hydrocolloïde adaptée pour adhérer à la peau.

13. Appareil selon l'une quelconque des revendications 1 à 12, dans lequel le corps allongé comporte une première extrémité (130) orientée vers le nez dans l'état monté et une deuxième extrémité opposée (132) orientée vers l'oreille dans l'état monté, et dans lequel le corps allongé est conçu pour se rétrécir vers la première extrémité afin de créer une partie de montage sur le nez (134) conformée pour être au moins partiellement complémentaire de la forme du nez à proximité d'une narine (7) de celui-ci afin de permettre le positionnement du corps allongé au moins partiellement en dessous et latéralement adjacent à la narine.

14. Procédé de retenue d'une sonde (11) sur le visage (13) d'un utilisateur (3) entre le nez (7) de l'utilisateur et une oreille (6) de l'utilisateur, le procédé comportant les étapes suivantes :
a. apposition d'un côté de fixation (129) d'un corps allongé (122) d'un support de sonde (121) sur le visage de telle façon que le corps allongé est sensiblement aligné entre le nez et l'oreille ;
b. placement d'une partie reçue (11F) de la sonde en utilisant un élément de placement (133) couplé au corps allongé et adapté pour recevoir la partie reçue de telle façon que la partie reçue de la sonde se trouve sensiblement dans le sens du corps allongé ;
c. déplacement d'un couvercle (128) couplé au corps allongé d'un état ouvert à un état fermé pour couvrir la sonde lorsque la sonde est reçue par l'élément de placement ;
d. positionnement d'une partie de montage sur le nez (134) du support de sonde au moins partiellement en dessous et latéralement adjacente à une narine (7) du nez de l'utilisateur, la partie de montage sur le nez étant conformée pour être au moins partiellement complémentaire de la forme du nez à proximité de la narine de celui-ci ; et
e. montage de la sonde sur l'élément de placement afin qu'elle s'étende depuis l'élément de placement à travers la partie de montage sur le nez et dans la narine du nez de l'utilisateur.
